# EUROPEAN PATENT APPLICATION

(11) **EP 2 719 404 A1**
(43) Date of publication of application: **16.04.2014**
(21) Application number: 12796246.2
(22) Date of filing: 08.06.2012
(51) Int. Cl.: A61M 1/14

(54) **BLOOD PURIFICATION DEVICE**

(30) Priority: 10.06.2011 JP 2011130280
(71) Applicant: Nikkiso Company Limited, Tokyo 150-6022 (JP)
(72) Inventor: FURUHASHI, Tomohiro, Makinohara-shi Shizuoka 421-0496 (JP); SUZUKI, Hiroaki, Makinohara-shi Shizuoka 421-0496 (JP); HIRANO, Takayuki, Makinohara-shi Shizuoka 421-0496 (JP); SUZUKI, Tomohiro, Makinohara-shi Shizuoka 421-0496 (JP)
(74) Representative: von Hirschhausen, Helge
(86) International application number: PCT/JP2012/064775
(87) International publication number: WO 2012/169610

(57) **Abstract**

[Object] To provide a blood purification apparatus which can easily and quickly find a cause for alarm issuing.

[Solution] The blood purification apparatus that performs blood purification treatment by purifying blood of a patient which is extracorporeally circulated and that includes a display device 2 which performs a display relating to the blood purification treatment. The display device 2 can switch and display a plurality of screens including an alarm screen displayed due to alarm issuing. The alarm screen is displayed together with multiple type information items required for finding a cause for the alarm issuing.

## Description

### Technical Field

The present invention relates to a blood purification apparatus that performs blood purification treatment by purifying blood of a patient which is extracorporeally circulated, including a display device which performs a display relating to the blood purification treatment.

### Background Art

A dialysis apparatus as a blood purification apparatus used in dialysis treatment is adapted such that a plurality of dialysis apparatuses are installed in a dialysis room disposed inside medical facilities such as hospitals and the dialysis treatment (blood purification treatment) is performed on a large number of patients inside the dialysis room. Such a dialysis apparatus generally has a display device arranged in order to perform a display relating to the dialysis treatment, and is configured to be capable of performing a predetermined operation relating to the dialysis treatment while viewing the display by using the display device. A plurality of screens displayed by using the display device are prepared in advance. Necessary information can be understood by switching the plurality of screens to be displayed.

Incidentally, a normal dialysis apparatus is configured to call attention by issuing an alarm when work relating to the dialysis treatment (operation for the dialysis treatment and operation for preparing the treatment) is carried out, when there is an operation mistake or erroneous setting, or for example, when parameters of a venous pressure which is monitored (monitored values) are beyond a preset range. Then, if such an alarm is issued, a display screen is automatically switched over to an alarm screen by the display device and items required for finding a cause for the alarm issuing are displayed for each item by using a text for example.

According to the dialysis apparatus in the related art, it is possible to display a desired screen required for finding the cause for the alarm issuing in such a manner that a health care worker such as a doctor operates to switch the screen of the display device or operates various operation devices such as an operation button in accordance with the text on the alarm screen displayed due to the alarm issuing, thereby enabling the dialysis apparatus to identify and deal with the cause for the alarm issuing. Since the related art does not pertain to the invention described in a publication, there is no information on related art documents to be described.

### Summary of Invention

### Technical Problem

However, in the above-described blood purification apparatus in the related art, in order to find and cope with the cause for the alarm issuing, it is necessary for the health care worker such as the doctor to operate switching of the screen of the display device or to operate various operation devices such as operation buttons in accordance with the text on the alarm screen. Therefore, the associated work is cumbersome and complicated, thereby causing a problem in that it takes time to release the alarm. In particular, in the blood purification apparatus, it is necessary to perform quick arrangement on the cause for the alarm issuing since there is a need to reduce burden on a patient. Therefore, shortening the time to release the alarm has been very strongly requested.

The present invention is made in view of such circumstances, and aims to provide a blood purification apparatus which can easily and quickly find a cause for alarm issuing.

### Solution to Problem

According to the invention described in Claim 1, there is provided a blood purification apparatus that performs blood purification treatment by purifying blood of a patient which is extracorporeally circulated, including a display device which performs a display relating to the blood purification treatment. The display device can switch and display a plurality of screens including an alarm screen displayed due to alarm issuing. The alarm screen is displayed together with multiple type information items required for finding a cause for the alarm issuing.

According to the invention described in Claim 2, in the blood purification apparatus described in Claim 1, in addition to a display of confirmation items required for finding the cause for the alarm issuing, the alarm screen displays at least any one of a numerical value of parameters of a monitoring subject relating to the blood purification treatment; a graphic indicating development of the parameters of the monitoring subject relating to the blood purification treatment; and a setting value relating to the blood purification treatment.

According to the invention described in Claim 3, in the blood purification apparatus described in Claim 1 or 2, the display on the alarm screen includes an image for indicating: the confirmation items required for finding the cause for the alarm issuing; and/or a coping method with respect to the cause for the alarm issuing.

According to the invention described in Claim 4, in the blood purification apparatus described in any one of Claims 1 to 3, the display device is a touch panel which enables a predetermined input by touching a switch unit displayed in a desired position on the screen. The alarm screen displays an operating switch unit which enables an operation in order to cope with the cause for the alarm issuing.

According to the invention described in Claim 5, in the blood purification apparatus described in Claim 4, the operating switch unit is a switch unit which enables an operation of stopping an alarm for a predetermined time period no matter if the cause for the alarm issuing has been released or not.

According to the invention described in Claim 6, in the blood purification apparatus described in Claim 4, the operating switch unit is a switch unit which enables an operation which directly copes with the cause for the alarm issuing.

According to the invention described in Claim 7, in the blood purification apparatus described in Claim 4, the alarm screen displays a shortcut switch unit which can switch to an operation screen enabling an operation to cope with the cause for the alarm issuing.

According to the invention described in Claim 8, in the blood purification apparatus described in any one of Claims 1 to 7, the display of the multiple type information items required for finding the cause for the alarm issuing can be individually set depending on a patient.

### Advantageous Effects of Invention

According to the invention described in Claim 1, the display device can switch and display the plurality of screens including the alarm screen displayed due to the alarm issuing. The alarm screen is displayed together with the multiple type information items required for finding the cause for the alarm issuing. Therefore, it is possible to easily and quickly find the cause for the alarm issuing.

According to the invention described in Claim 2, in addition to the display of the confirmation items required for finding the cause for the alarm issuing, the alarm screen displays at least any one of the numerical value of the parameters of the monitoring subject relating to the blood purification treatment; the graphic indicating development of the parameters of the monitoring subject relating to the blood purification treatment; and the setting value relating to the blood purification treatment. Therefore, it is possible to more accurately find the cause for the alarm issuing.

According to the invention described in Claim 3, the display on the alarm screen includes the image for indicating: the confirmation items required for finding the cause for the alarm issuing; and/or the coping method with respect to the cause for the alarm issuing, thereby enabling the coping method to be visually checked. Therefore, it is possible to more easily and accurately use the coping method required for finding the cause for the alarm issuing.

According to the invention described in Claim 4, the display device is the touch panel which enables the predetermined input by touching the switch unit displayed in the desired position on the screen. The alarm screen displays the operating switch unit which enables the operation in order to cope with the cause for the alarm issuing. Therefore, it is possible to eliminate a need for an operation device separated from the display device, thereby enabling a configuration of the blood purification apparatus to be simplified.

According to the invention described in Claim 5, the operating switch unit is the switch unit which enables the operation of stopping the alarm for the predetermined time period no matter whether or not the cause for the alarm issuing has been released. Therefore, it is possible to more quickly perform the operation for temporary alarm releasing.

According to the invention described in Claim 6, the operating switch unit is the switch unit which enables the operation which directly copes with the cause for the alarm issuing. Therefore, it is possible to more quickly perform the operation for the alarm releasing.

According to the invention described in Claim 7, the alarm screen displays the shortcut switch unit which can switch to the operation screen enabling the operation to cope with the cause for the alarm issuing. Therefore, even a person who is unfamiliar with the switching operation of the display device can smoothly switch to the operation screen for the alarm releasing.

According to the invention described in Claim 8, the display of the multiple type information items required for finding the cause for the alarm issuing with respect to the alarm screen can be individually set depending on the patient. Therefore, it is possible to display the alarm screen conforming to a condition of each patient.

### Brief Description of Drawings

[Fig. 1] Fig. 1 is a front view illustrating a blood purification apparatus according to an embodiment of the present invention.
[Fig. 2] Fig. 2 is a conceptual diagram illustrating a screen configuration displayed on a display device in the blood purification apparatus.
[Fig. 3] Fig. 3 is a schematic diagram illustrating an alarm screen (main alarm screen) displayed on the display device.
[Fig. 4] Fig. 4 is a schematic diagram illustrating an alarm screen (de tailed alarm screen) displayed on the display device.
[Fig. 5] Fig. 5 is a schematic diagram illustrating an alarm screen (main alarm screen) displayed on the display device.
[Fig. 6] Fig. 6 is a schematic diagram illustrating an alarm screen (de tailed alarm screen) displayed on the display device.
[Fig. 7] Fig. 7 is a schematic diagram illustrating an alarm screen (main alarm screen) displayed on the display device.
[Fig. 8] Fig. 8 is a schematic diagram illustrating an alarm screen (de tailed alarm screen) displayed on the display device.

### Description of Embodiments

Hereinafter, an embodiment of the present invention will be described in detail with reference to the drawings.

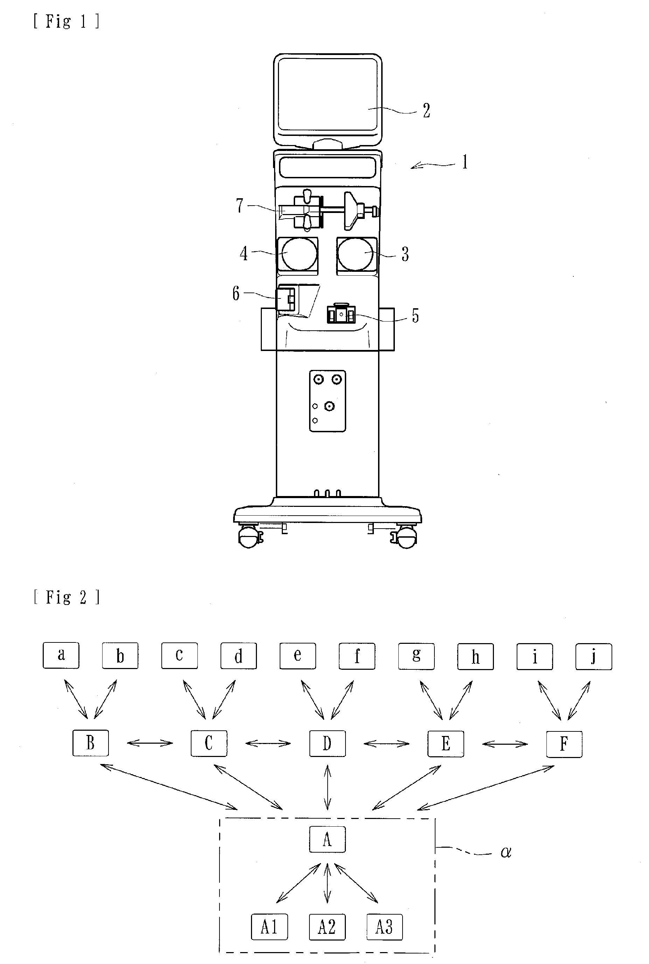

A blood purification apparatus according to the present embodiment is applied to a hemodialysis apparatus that performs dialysis treatment by purifying blood of a patient which is extracorporeally circulated. As illustrated in Fig. 1, the blood purification apparatus is mainly configured to include a dialysis device 1 (dialysis monitoring device) having a blood pump 3, a substitution pump 4, a bypass connector receiver 5, a bubble detector 6 and an infusion pump 7; and a display device 2 which is arranged above the dialysis device 1 and performs a display relating to blood purification treatment.

In the dialysis device 1, pipes such as a dialysate introduction line and a dialysate discharge line (not illustrated) are formed. A dialyzer (blood purifier) is connected to the dialysate introduction line and the dialysate discharge line, and a blood circuit is connected to the dialyzer. Then, the blood circuit can extracorporeally circulate the blood of the patient and the dialyzer can purify the blood which is extracorporeally circulated.

The blood pump 3 is attached to the blood circuit (artery side blood circuit) and causes the blood of the patient to flow through a flexible tube configuring the blood circuit. The substitution pump 4 causes a substitution to flow inside the blood circuit through a tube for the substitution. In addition, the bubble detector 6 detects a bubble inside the blood circuit. The infusion pump 7 drives a syringe containing an anticoagulant (heparin) for example and continuously infuses the anticoagulant into the blood circuit.

In addition, the bypass connector receiver 5 receives and holds a bypass connector. When the blood purification treatment is not performed, the bypass connector is connected to a tip of the dialysate introduction line (dialysate exit port) and a tip of the dialysate discharge line (dialysate returning port), and can short-circuit (connects the tips to each other) the dialysate introduction line and the dialysate discharge line. Then, when short-circuiting the dialysate introduction line and the dialysate discharge line, the bypass connector is held by the bypass connector receiver 5. Therefore, if the bypass connector is held by the bypass connector receiver 5, a switch reacts, thereby enabling detection that the dialysate introduction line and the dialysate discharge line are short-circuited.

The display device 2 is a touch panel (for example, touch panel such as a color LCD) which enables a predetermined input by touching a switch unit displayed in a desired position on a screen. The display device 2 can switch and display a plurality of screens including an alarm screen displayed due to alarm issuing. More specifically, as illustrated in Fig. 2, the display device 2 can switch and display screens A to F. For example, when the screen B is displayed in a standby state before blood purification treatment, the display device 2 displays an operation screen which enables a predetermined operation through an input operation by touching a predetermined switch unit displayed on the screen B. Alternatively, when the screen C is displayed during the blood purification treatment, the display device 2 can switch to a screen d displaying a numerical value of parameters of a monitoring subject relating to the blood purification treatment or a screen d displaying a graphic indicating development of the parameters of the monitoring subject relating to the blood purification treatment through the input operation by touching a predetermined switch unit displayed on the screen C.

That is, on the screen switched and displayed on the display device 2, various information items relating to the blood purification treatment (various setting values during the blood purification treatment, monitored values indicating current values of the parameters of the monitoring subject, or graphics indicating time-dependent variations) are displayed. Based on these information items, the treatment can be performed. In this manner, in addition to the display relating to the blood purification treatment (for example, a variation rate of circulating blood volume (ΔBV) which is an index indicating a condition of a patient under treatment or a graphic display indicating the time-dependent variations in the venous pressure), the display device 2 according to the present embodiment enables an input operation for setting or changing the parameters relating to the treatment (for example, drive speed of the blood pump 3 or drive speed of ultrafiltration pump (not illustrated)).

During dialysis treatment (blood purification treatment), the display device 2 according to the present embodiment can display the following items. That is, the display device 2 can display icons for various operations, such as an operation status (in a HD operation), various pressure values (venous pressure and dialysate pressure), ultrafiltration conditions (ultrafiltration volume integration, ultrafiltration volume setting and ultrafiltration speed), dialysate conditions (dialysate temperature, dialysate concentration and dialysate flow rate), an anticoagulant infusion speed (IP speed), a blood flow rate, elapsed time of dialysis treatment, and blood pressure/pulse values.

Here, in the present embodiment, an alarm is issued to call attention if the blood purification apparatus detects an abnormality, such as when work relating to the dialysis treatment (operation for the dialysis treatment and operation for preparing the treatment) is carried out, when there is an operation mistake or erroneous setting, or for example, when parameters of the venous pressure and ΔBV which are monitored (monitored values) are beyond a preset range. Then, if the alarm is issued, the display on the display device 2 is automatically switched over to an alarm screen α (in the present embodiment, a main alarm screen A first appears). A device for issuing the alarm may have any form, either those which output an alarm sound using a speaker or those which turn on an alarm lamp.

First, a case will be described where the alarm is issued since the venous pressure monitored by the blood purification apparatus (venous pressure detected based on a pressure of an air layer in an air trap chamber of a venous side blood circuit) is beyond the preset range (beyond an upper limit value or a lower limit value).

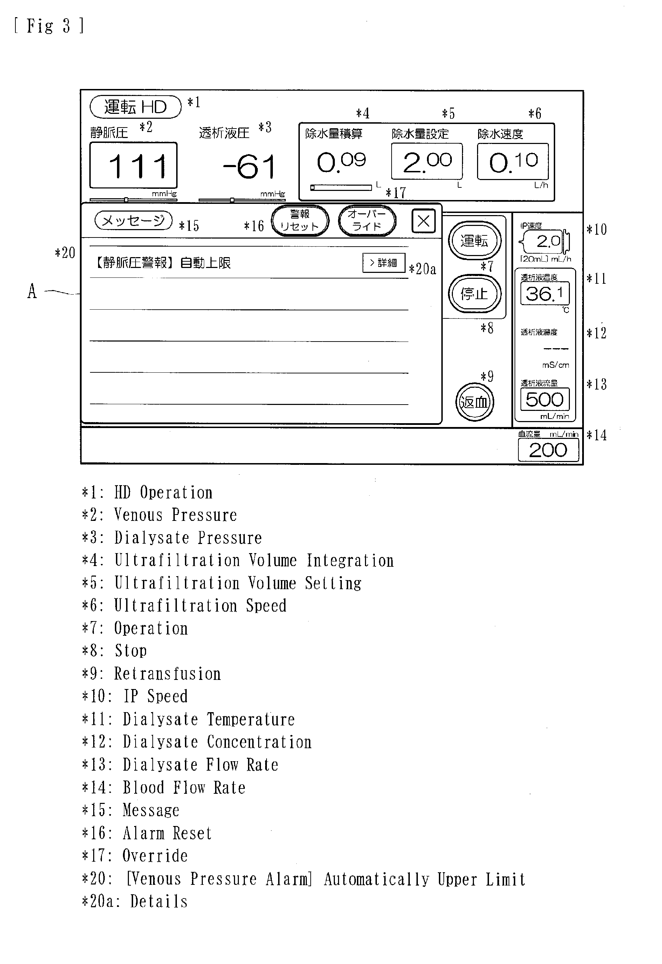

If the alarm is issued since the venous pressure monitored by the blood purification apparatus is beyond the preset range, the screen on the display device 2 is automatically switched over to the main alarm screen A as illustrated in Fig. 3. As illustrated in Fig. 3, the main alarm screen A displays a cause for the alarm issuing (in this case, assuming a cause where the venous pressure is beyond the preset range) in text. The main alarm screen A continuously performs the various information displays before the alarm is issued (displays of the venous pressure, the dialysate pressure, the ultrafiltration volume integration, the ultrafiltration volume setting, the ultrafiltration speed, the IP speed (continuous infusion speed from a syringe using the infusion pump 7), the dialysate temperature, the dialysate flow rate, and the operation or stoppage of the dialysis apparatus, or display of the switch unit for the retransfusion).

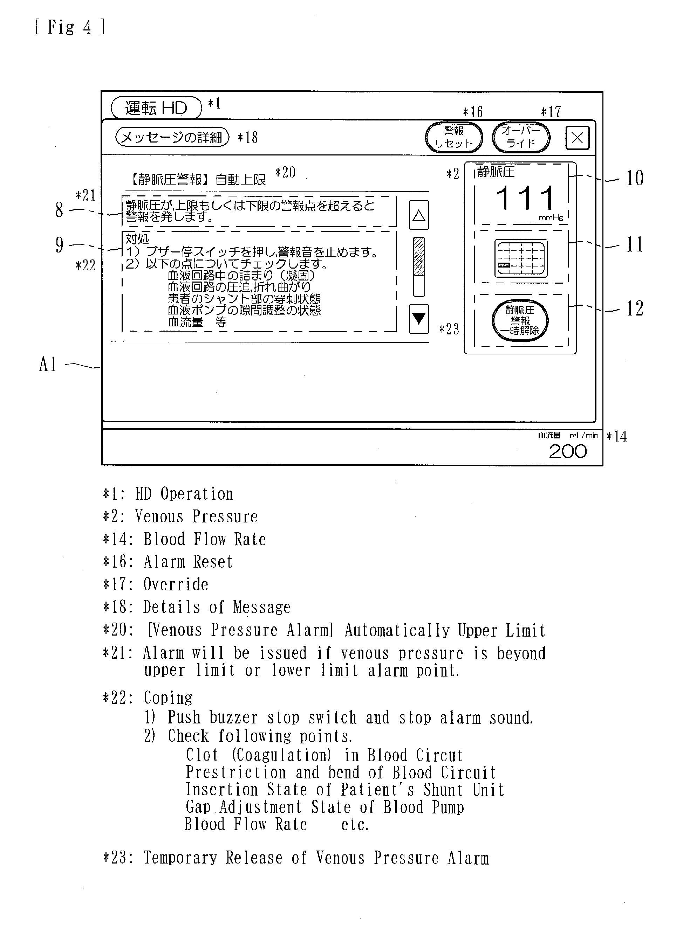

Then, if the input operation is performed by touching the switch unit displayed on the main alarm screen A as "details", the display using the display device 2 is switched over to a detailed alarm screen A1 as illustrated in Fig. 4. The detailed alarm screen A1 displays information together with multiple type information items required for finding the cause for the alarm issuing. In the present embodiment, the detailed alarm screen A1 displays a cause explanation unit 8 indicating items which can be considered as the cause for the alarm; a confirmation item unit 9 indicating confirmation items required for finding the cause for the alarm issuing; a monitored value unit 10 indicating a numerical value of the parameters of the monitoring subject relating to the blood purification treatment (in this case, the monitored value of the venous pressure because it is the alarm relating to an abnormality of the venous pressure); and a graphic unit 11 indicating a graphic illustrating development of the parameters of the monitoring subject relating to the blood purification treatment (in this case, the graphic illustrating the development of the venous pressure because it is the alarm relating to the abnormality of the venous pressure).

Then, in the detailed alarm screen A1 as described above, the multiple type information items required for finding the cause for the alarm issuing (specifically, the cause explanation unit 8, the confirmation item unit 9, the monitored value unit 10 and the graphic unit 11) are displayed together on an identical screen. In this manner, it is possible to visually and simultaneously check and diversely understand the multiple type information items relating to the alarm. Accordingly, a health care worker such as a doctor can easily find the cause for the alarm issuing. In the detailed alarm screen A1, as long as the multiple type information items required for finding the alarm issuing are displayed together on the identical screen, any of the cause explanation unit 8, the confirmation item unit 9, the monitored value unit 10 and the graphic unit 11 may be arbitrarily combined and selectively displayed. Alternatively, in addition to the cause explanation unit 8, the confirmation item unit 9, the monitored value unit 10 and the graphic unit 11, the other type information items relating to the alarm may be displayed.

Further, in addition to the cause explanation unit 8, the confirmation item unit 9, the monitored value unit 10 and the graphic unit 11, the detailed alarm screen A1 displays an operating switch unit 12 which enables an operation for coping with the cause for the alarm issuing. The operating switch unit 12 illustrated in Fig. 4 is a switch unit which enables the operation of stopping the alarm for a predetermined time period no matter whether or not the cause for the alarm issuing has been released. After temporarily stopping the alarm by operating the operating switch unit 12, it is possible to find the cause for the alarm issuing or to cope with the cause for the alarm issuing when necessary.

In this manner, in addition to the displays of the cause explanation unit 8 indicating items which can be considered as the cause for the alarm and the confirmation items (confirmation item unit 9) required for finding the cause for the alarm issuing, the detailed alarm screen A1 displays the numerical value of the parameters of the monitoring subject relating to the blood purification treatment (monitored value unit 10) and the graphic illustrating development of the parameters of the monitoring subject relating to the blood purification treatment (graphic unit 11). Therefore, it is possible to simultaneously display multiple different type information items relating to the alarm, and thus it is possible to more accurately find the cause for the alarm issuing. In particular, the operating switch unit 12 is a switch unit which enables the operation of stopping the alarm for the predetermined time period no matter whether or not the cause for the alarm issuing has been released. Therefore, it is possible to more quickly perform the operation for the temporary alarm releasing.

Next, a case will be described where the alarm is issued due to erroneous setting of the infusion pump 7.

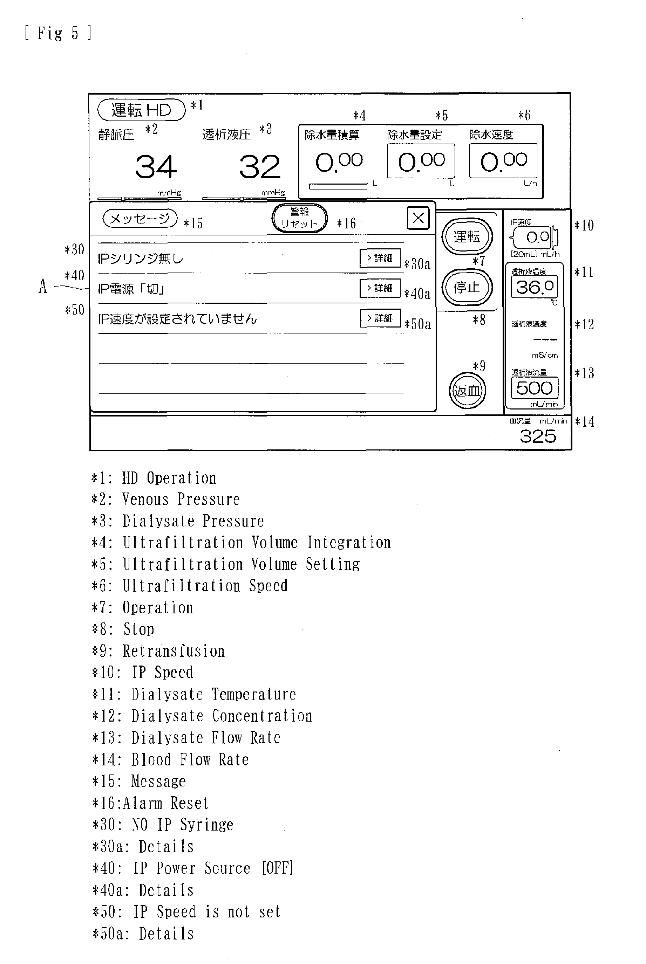

If the alarm is issued due to the erroneous setting of the infusion pump 7, the screen of the display device 2 is automatically switched over to the main alarm screen A as illustrated in Fig. 5. As illustrated in Fig. 5, the main alarm screen A displays the cause for the alarm issuing in text (in this case, indication that the IP syringe (syringe) is not set, indication that the IP power source is in a disconnected state (that is, OFF), or indication that the IP speed (continuous infusion speed from the syringe using the infusion pump 7) is not set). The main alarm screen A continuously performs the various information displays before the alarm is issued (displays of the venous pressure, the dialysate pressure, the ultrafiltration volume integration, the ultrafiltration volume setting, the ultrafiltration speed, the IP speed (continuous infusion speed from a syringe using the infusion pump 7), the dialysate temperature, the dialysate flow rate, and the operation or stoppage of the dialysis apparatus, or display of the switch unit for the retransfusion).

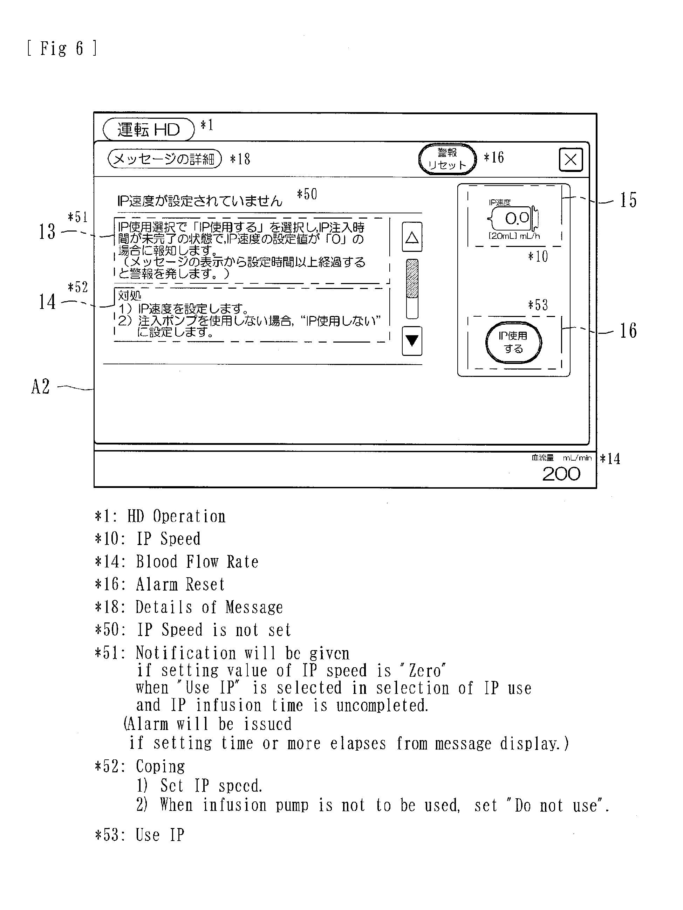

Then, if the input operation is performed by touching the switch unit (for example, the switch unit having a character display that "the IP speed is not set") displayed on the main alarm screen A as "details", the display using the display device 2 is switched over to a detailed alarm screen A2 as illustrated in Fig. 6. The detailed alarm screen A2 displays information together with multiple type information items required for finding the cause for the alarm issuing. In the present embodiment, the detailed alarm screen A2 displays a cause explanation unit 13 indicating items which can be considered as the cause for the alarm; a confirmation item unit 14 indicating confirmation items required for finding the cause for the alarm issuing; and a setting value unit 15 indicating a setting value relating to the blood purification treatment (in this case, the setting value of the IP speed because it is the alarm relating to the IP speed).

In the detailed alarm screen A2 as described above, the multiple type information items required for finding the cause for the alarm issuing (specifically, the cause explanation unit 13, the confirmation item unit 14 and the setting value unit 15) are displayed together on an identical screen. In this manner, it is possible to visually and simultaneously check and diversely understand the multiple type information items relating to the alarm. Accordingly, a health care worker such as a doctor can easily find the cause for the alarm issuing. In the detailed alarm screen A2, as long as the multiple type information items required for finding the alarm issuing are displayed together on the identical screen, in addition to the cause explanation unit 13, the confirmation item unit 14 and the setting value unit 15, the other type information items relating to the alarm may be displayed.

Further, in addition to the cause explanation unit 13, the confirmation item unit 14 and the setting value unit 15, the detailed alarm screen A2 displays an operating switch unit 16 which enables an operation for coping with the cause for the alarm issuing. The operating switch unit 16 illustrated in Fig. 6 is a switch unit which enables the operation for directly coping with the cause for the alarm issuing. It is possible to achieve the alarm releasing by operating the operating switch unit 16, for example, by changing the setting which indicates that the IP will not be used (that is, the continuous infusion of the anticoagulant will not be performed by using the syringe). The operating switch may be sufficiently configured if the operating switch unit is a switch unit which enables the operation for directly coping with the cause for the alarm issuing, and for example, may be those which can change the setting value of the blood flow rate or the dialysate flow rate.

In this manner, in addition to the cause explanation unit 13 indicating the items which can be considered as the cause for the alarm and the display of the confirmation items (confirmation item unit 14) required for finding the cause for the alarm issuing, the detailed alarm screen A2 displays the setting value relating to the blood purification treatment (setting value unit 15). Therefore, it is possible to simultaneously display the multiple different type information items relating to the alarm, and thus it is possible to more accurately find the cause for the alarm issuing. In particular, the operating switch unit 16 is the switch unit which enables the operation for directly coping with the cause for the alarm issuing. Therefore, it is possible to more quickly perform the operation for the alarm releasing.

Next, a case will be described where the operation of the blood purification apparatus is stopped and the alarm is issued.

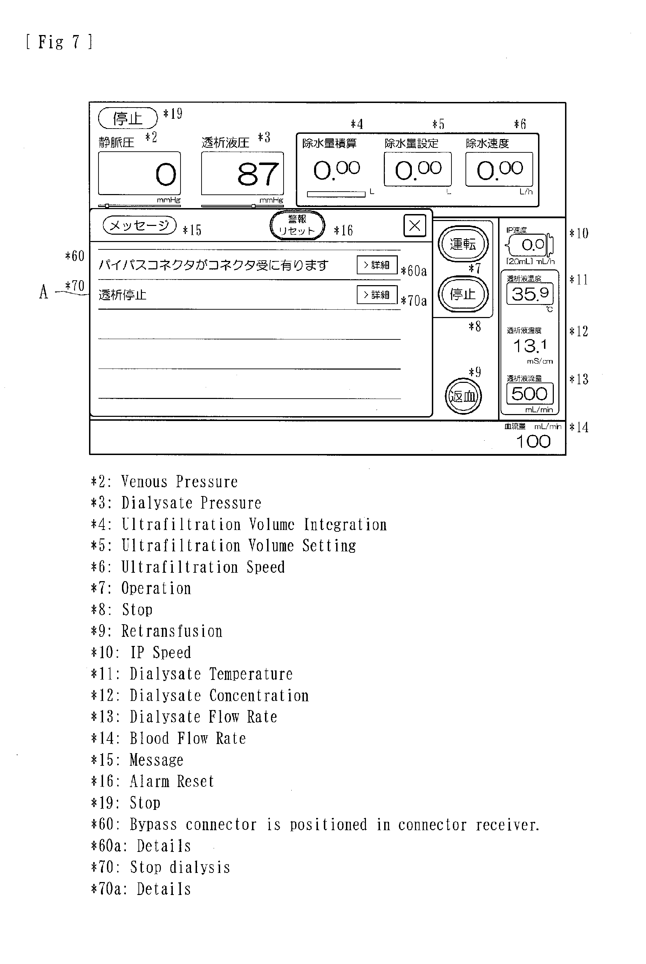

If the operation of the blood purification apparatus is stopped and the alarm is issued, the screen of the display device 2 is automatically switched over to the main alarm screen A as illustrated in Fig. 7. As illustrated in Fig. 7, the main alarm screen A displays the cause for the alarm issuing in text (in this case, indication that the bypass connector is positioned in the connector receiver 5, and indication that the dialysis is stopped (that is, the dialysis treatment is stopped)). The main alarm screen A continuously performs the various information displays before the alarm is issued (displays of the venous pressure, the dialysate pressure, the ultrafiltration volume integration, the ultrafiltration volume setting, the ultrafiltration speed, the IP speed (continuous infusion speed from a syringe using the infusion pump 7), the dialysate temperature, the dialysate flow rate, and the operation or stoppage of the dialysis apparatus, or display of the switch unit for the retransfusion).

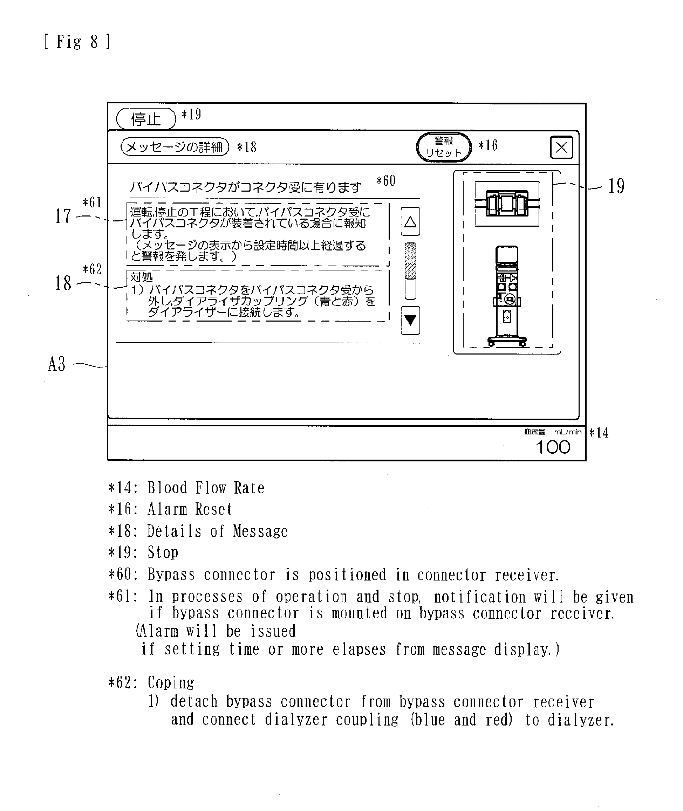

Then, if the input operation is performed by touching the switch unit (for example, the switch unit having a character display that "the bypass connector is positioned in the connector receiver") displayed on the main alarm screen A as "details", the display using the display device 2 is switched over to a detailed alarm screen A3 as illustrated in Fig. 8. The detailed alarm screen A3 displays information together with multiple type information items required for finding the cause for the alarm issuing. Therefore, in the present embodiment, the detailed alarm screen A3 displays a cause explanation unit 17 indicating items which can be considered as the cause for the alarm; a confirmation item unit 18 indicating confirmation items required for finding the cause for the alarm issuing; and an image 19 for indicating confirmation items required for finding the cause for the alarm issuing or a coping method with respect to the cause for the alarm issuing. For example, the image 19 shows the entire blood purification apparatus (dialysis apparatus) by using an image, and enables the cause for the alarm issuing or a portion relating to the finding of the cause for the alarm issuing to be specified and enables the details to be visually checked. Therefore, the image 19 may be either a still image or a video image.

Then, in the detailed alarm screen A3 as described above, the multiple type information items required for finding the cause for the alarm issuing (specifically, the cause explanation unit 17, the confirmation item unit 18 and the image 19) are displayed together on an identical screen. In this manner, it is possible to visually and simultaneously check and diversely understand the multiple type information items relating to the alarm. Accordingly, a health care worker such as a doctor can easily find the cause for the alarm issuing. In the detailed alarm screen A3, as long as the multiple type information items required for finding the alarm issuing are displayed together on the identical screen, in addition to the cause explanation unit 17, the confirmation item unit 18 and the image 19, the other type information items relating to the alarm may be displayed.

In this manner, in addition to the cause explanation unit 17 indicating the items which can be considered as the cause for the alarm and the display of the confirmation items (confirmation item unit 18) required for finding the cause for the alarm issuing, the detailed alarm screen A3 displays the image 19 for indicating the confirmation items required for finding the cause for the alarm issuing or the coping method with respect to the cause for the alarm issuing. Therefore, it is possible to simultaneously display the multiple different type information items relating to the alarm, and thus it is possible to more accurately find the cause for the alarm issuing. In particular, the display of the detailed alarm screen A3 includes the image for the confirmation items required for finding the cause for the alarm issuing or the coping method with respect to the cause for the alarm issuing. Therefore, it is possible to visually understand the confirmation items or the coping method, and thus it is possible to more easily and accurately use the confirmation required for finding the cause for the alarm issuing or the coping method.

According to the present embodiment, the display device 2 can switch and display the plurality of screens including the alarm screen α displayed due to the alarm issuing. The alarm screen α (in the present embodiment, the detailed alarm screen) displays the information together with the multiple type information items required for finding the cause for the alarm issuing. Accordingly, a health care worker such as a doctor can diversely understand the information relating to the alarm, and thus it is possible to easily and quickly find the cause for the alarm issuing. According to the present embodiment, in the alarm screen, whereas the multiple type information items required for finding the cause for the alarm issuing are displayed together, the other information which does not relate to the alarm is not displayed. In this manner, a worker can concentrate his or her senses on finding or coping with the cause for the alarm issuing.

Hitherto, the present embodiment has been described, but the present invention is not limited thereto. For example, the screen of the display device may be set to exclusively perform the display and a mechanical switch or the like may be arranged near the screen so as to enable the input operation. However, if the display device 2 as in the present embodiment is the touch panel which enables a predetermined input by touching the switch unit displayed in a desired position on the screen, and if the alarm screen displays the operating switch unit which enables an operation to cope with the cause for the alarm issuing, it is possible to eliminate a need for an operating device separated from the display device 2, thereby enabling a configuration of the blood purification apparatus to be simplified.

In addition, the main alarm screen in the alarm screen α may be omitted and if the alarm is issued, the detailed alarm screen may be directly switched over to be displayed. Alternatively, other displays such as the number of the alarms issued may be added to the display on the alarm screen. In addition, the alarm screen performs the display as described in the above embodiment, and may display the shortcut switch unit which can switch to the operation screen capable of performing the operation for coping with the cause for the alarm issuing. In this case, even a person who is unfamiliar with the switching operation of the display device 2 can smoothly switch to the operation screen for the alarm releasing.

Furthermore, the display of the multiple type information items required for finding the cause for the alarm issuing can be individually set depending on the patient. For example, for a dynamically moving patient, a patient constantly having a high venous pressure or a patient constantly having a low venous pressure, it is possible to change the alarm screen depending on the patient's inherent conditions (types of displays, the layout and size of each display are change depending on the patient). In this case, it is possible to display the alarm screen which matches the condition for each patient.

In addition, in the present embodiment, the display device 2 is applied to the hemodialysis apparatus in a hemodialysis (HD). However, instead of this, the display device 2 may be applied to a blood purification apparatus which can perform the other blood purification treatment (for example, treatment using hemodiafiltration (HDF), hemofiltration (HF) or slow continuous hemodiafiltration (CHF)).

### Industrial Applicability

If a display device can switch and display a plurality of screens including an alarm screen displayed due to alarm issuing, and if a blood purification apparatus allows the alarm screen to display information together with multiple type information items required for finding a cause for the alarm issuing, the display device can be applied to those whose outer shape is different or those which have the other added functions.

### Reference Signs List

- 1: dialysis device
- 2: display device (touch panel)
- 3: blood pump
- 4: substitution pump
- 5: bypass connector receiver
- 6: bubble detector
- 7: infusion pump
- 8: cause explanation unit
- 9: confirmation item unit
- 10: monitored value unit
- 11: graphic unit
- 12: operating switch unit
- 13: cause explanation unit
- 14: confirmation item unit
- 15: setting value unit
- 16: operating switch unit
- 17: cause explanation unit
- 18: confirmation item unit
- 19: image
- α: alarm screen
- A1: alarm screen (main alarm screen)
- A2: alarm screen (detailed alarm screen)

## Claims

1. A blood purification apparatus that performs blood purification treatment by purifying blood of a patient which is extracorporeally circulated, com prising:
a display device which performs a display relating to the blood purification treatment,
wherein the display device can switch and display a plurality of screens including an alarm screen displayed due to alarm issuing, and
wherein the alarm screen is displayed together with multiple type infomation items required for finding a cause for the alarm issuing.

2. The blood purification apparatus according to Claim 1,
wherein in addition to a display of confirmation items required for finding the cause for the alarm issuing, the alarm screen displays at least any one of a numerical value of parameters of a monitoring subject relating to the blood purification treatment; a graphic indicating development of the parameters of the monitoring subject relating to the blood purification treatment; and a setting value relating to the blood purification treatment.

3. The blood purification apparatus according to Claim 1 or 2,
wherein the display on the alarm screen includes an image for indicating: the confirmation items required for finding the cause for the alarm issuing; and/or a coping method with respect to the cause for the alarm issuing.

4. The blood purification apparatus according to any one of Claims 1 to 3,
wherein the display device is a touch panel which enables a predetermined input by touching a switch unit displayed in a desired position on the screen, and
wherein the alarm screen displays an operating switch unit which enables an operation in order to cope with the cause for the alarm issuing.

5. The blood purification apparatus according to Claim 4,
wherein the operating switch unit is a switch unit which enables an operation of stopping an alarm for a predetermined time period no matter whether or not the cause for the alarm issuing has been released.

6. The blood purification apparatus according to Claim 4,
wherein the operating switch unit is a switch unit which enables an operation which directly copes with the cause for the alarm issuing.

7. The blood purification apparatus according to Claim 4,
wherein the alarm screen displays a shortcut switch unit which can switch to an operation screen enabling an operation to cope with the cause for the alarm issuing.

8. The blood purification apparatus according to any one of Claims 1 to 7,
wherein the display of the multiple type information items required for finding the cause for the alarm issuing can be individually set depending on a patient.
